# EUROPEAN PATENT SPECIFICATION

(11) **EP 2 978 468 B1**
(45) Date of publication and mention of the grant of the patent: **13.11.2019**
(21) Application number: 14715086.6
(22) Date of filing: 27.03.2014
(51) Int. Cl.: A61M 1/16

(54) **DISPOSABLE CARTRIDGE SYSTEM FOR USE WITH SORBENT OR PREMIXED DIALYSATE**
EINWEGKARTUSCHENSYSTEM ZUR VERWENDUNG MIT SORPTIONSMITTEL ODER VORGEMISCHTEM DIALYSAT
SYSTÈME DE CARTOUCHE JETABLE DEVANT ÊTRE UTILISÉ AVEC UN SORBANT OU UN DIALYSAT PRÉALABLEMENT MÉLANGÉ

(30) Priority: 28.03.2013 GB 201305757
(43) Date of publication of application: 03.02.2016
(73) Proprietor: Quanta Dialysis Technologies Limited, Alcester Warwickshire B49 6EU (GB)
(72) Inventor: BUCKBERRY, Clive, Warwick Warwickshire CV34 6AH (GB)
(74) Representative: Range, Christopher William
(86) International application number: PCT/GB2014/050979
(87) International publication number: WO 2014/155121

(56) References cited:
- WO-A1-2011/017215
- WO-A2-2005/044339
- WO-A2-2010/042666
- WO-A2-2013/019994
- DE-A1- 3 110 128
- US-A1- 2004 019 312
- US-A1- 2009 008 306
- US-A1- 2009 127 193
- US-A1- 2009 198 170

## Description

The present invention relates to dialysis machines and in particular, but not exclusively to a disposable cartridge for use in hemodialysis machines.

Dialysis is a treatment which replaces the renal function of removing excess fluid and waste products, such as potassium and urea, from blood. The treatment is either employed when renal function has deteriorated to an extent that uremic syndrome becomes a threat to the body's physiology (acute renal failure) or, when a longstanding renal condition impairs the performance of the kidneys (chronic renal failure).

There are two major types of dialysis, namely hemodialysis and peritoneal dialysis.

In peritoneal dialysis treatment, a dialysate solution is run through a tube into the peritoneal cavity. The fluid is left in the cavity for a period of time in order to absorb the waste products, and is subsequently removed through the tube for disposal.

It is common for patients in the early stages of treatment for a longstanding renal condition to be treated by peritoneal dialysis before progressing to hemodialysis at a later stage.

In hemodialysis, the patient's blood is removed from the body by an arterial line and treated by a dialysis machine before being returned to the patient's body by a venous line. The machine passes the blood through a dialyser containing tubes formed from a semi-permeable membrane. On the exterior of the semi-permeable membrane is a dialysate solution. The semi-permeable membrane filters the waste products and excess fluid from the blood into the dialysate solution. The membrane allows the waste and a controlled volume of fluid to permeate into the dialysate solution whilst preventing the loss of larger more desirable molecules, like blood cells and certain proteins and polypeptides.

The action of dialysis across the membrane is achieved primarily by diffusion (the migration of molecules by random motion from a region of higher concentration to a region of lower concentration).

Fluid removal (otherwise known as ultrafiltration) is achieved by altering the hydrostatic pressure of the dialysate solution side of the membrane, causing free water to move across the membrane along the pressure gradient.

The correction of uremic acidosis of the blood is achieved by use of a bicarbonate buffer. The bicarbonate buffer also allows the correction of the blood bicarbonate level.

The dialysate solution consists of a sterilized solution of mineral ions. These ions are contained within an acid buffer which is mixed with the purified water and bicarbonate base prior to delivery to the dialyser.

The composition of the dialysate solution needs to be tightly controlled to keep the patient's blood at an optimal composition. Typically, the dialysate solution is passed through the dialyser once before being discarded to ensure that the composition of the dialysate solution remains constant. A single dialysis session takes four hours with approximately 120 litres of dialysate solution being used per session. Because dialysis machines are large, they require a significant volume of water purified by reverse osmosis per session and are expensive to purchase. Patients being treated for a renal condition are typically required to either attend a medical facility for each session or, in some cases, self dialyse at home. Typical hemodialysis machines are the size of a conventional refrigerator and as such cannot be transported without a suitable vehicle such as a van.

WO 2011/017215 discloses dialysis systems, components and methods, US 2009/198170 discloses a multi-pass dialysis system, US 2009/008306 discloses an extracorporeal dialysis ready peritoneal dialysis machine, WO 2005/044339 discloses a high convection home hemodialysis/hemofiltration and sorbent system, WO 2010/042666 discloses a priming system and method for dialysis systems, US 2004/019312 discloses systems and methods for performing peritoneal dialysis, and US 2009/127193 discloses dialysis systems and methods.

The present invention seeks to provide an improved hemodialysis machine. The invention is defined by the features of independent claim 1.

A first aspect of the invention provides a hemodialysis machine defining a closed loop fluid path for dialysate solution, the fluid path comprising: a first pump chamber having a pump inlet and a pump outlet, a dialyser having a dialyser inlet and a dialyser outlet, a conduit connected between the outlet of the first pump chamber and the inlet of the dialyser, a second pump chamber having a pump inlet and a pump outlet, a conduit connected between the outlet of the dialyser and the inlet of the second pump chamber, a dialysate regeneration device having an inlet and an outlet, a conduit connected between the outlet of the second pump chamber and the inlet of the dialysate regeneration device, a reservoir having a reservoir inlet and a reservoir outlet, a conduit connected between the outlet of the dialysate regeneration device and the inlet of the reservoir, and, a conduit connected between the outlet of the reservoir and the inlet of the first pump chamber, characterized in that the reservoir comprises a housing defining a continuous side wall, a top, a bottom, the inlet of the reservoir and the first outlet of the reservoir, and an overflow device mounted therein.

Advantageously, filtering spent dialysate solution significantly reduces the amount of dialysate solution required during a single dialysis session from 120 litres to in the region of 6 to 10 litres. The reduced amount of dialysate solution required can be provided in a pre-mixed supply thus removing the requirement to connect the hemodialysis machine to a permanent source of purified water, for example water purified by reverse osmosis from a mains supply.

In one embodiment, the dialyser comprises a cylindrical housing defining said dialyser inlet and said dialyser outlet therein, said dialyser inlet and said dialyser outlet being disposed substantially orthogonal to the longitudinal axis of the housing.

Preferably, the dialyser further comprises a semi-permeable membrane disposed within the housing, the membrane extending substantially between a first end of said housing and a second end of said housing. One side of the membrane is, in use, exposed to the dialysate solution and the other side to blood from the patient. The dialyser is a conventional component of dialysis machines.

In one embodiment, the hemodialysis machine further comprises a third pump chamber having a pump inlet and a pump outlet, the third pump chamber being connected between the reservoir and the first pump chamber inlet.

In one embodiment, the hemodialysis machine further comprises a fourth pump chamber having a pump inlet and a pump outlet, the fourth pump chamber being connected between the third pump chamber and the first pump chamber.

In one embodiment, the hemodialysis machine further comprises a fifth pump chamber having a pump inlet and a pump outlet, the fifth pump chamber being connected between the first pump chamber and the second pump chamber.

In one embodiment, the first, second, third, fourth and fifth pump chambers each include a respective flexible diaphragm, thus defining respective first, second, third and fourth pumps.

Preferably, the first and second pumps are flow balance pumps. In the invention the first and second pumps are of approximately equal capacity but due to variations in manufacture cannot be assumed to be of identical capacity. During hemodialysis treatment it is critical that the amount of fluid drawn from a patient as waste product is replaced by an equal amount of fluid from the dialysate solution. As will be described further below, the first and second pumps can either pump clean dialysate solution to the dialyser or receive spent dialysate solution from the dialyser. To ensure that the amount of fluid pumped to the dialyser is equal, to within clinical tolerances, to the amount of fluid received from the dialyser, the function of the first and second flow balance pumps is reversed approximately every 20 cycles of operation.

In one embodiment, each of the first, second, third, fourth and fifth pump chambers are provided on a cartridge removably mountable in the hemodialysis machine.

Provision of a cartridge as a consumable component, in combination with filtration of dialysate solution, allows for the hemodialysis machine to be optimised for convenient transportation with the patient. This is advantageous as typically a patient is required to be treated either at a medical facility or at home due to the large size of conventional hemodialysis machines. A smaller machine, similar to the size of a conventional desktop computer, would permit a patient to self-dialyse at any convenient location.

Preferably, the cartridge is a disposable cartridge.

The cartridge of the invention permits the majority of the fluid components of the hemodialysis machine to be disposed of after each dialysis session, thus reducing the risk of contamination or infection.

In one embodiment, the cartridge further comprises: a first inlet port, a conduit between the first inlet port and the inlet of the third pump chamber, a first outlet port, a conduit between the first inlet port and the first outlet port, a second inlet port, a conduit between the second inlet port and the inlet of the third pump chamber, a conduit between the outlet of the third pump chamber and the inlet of the fourth pump chamber, a third inlet port, a conduit between the third inlet port and the inlet of the fourth pump chamber, a conduit between the outlet of the fourth pump chamber and the inlet of the first pump chamber, a second outlet port, a conduit between the outlet of the first pump chamber and the second outlet port, a fourth inlet port, a conduit between the fourth inlet port and the inlet of the second pump chamber, a third outlet port, and, a conduit between the outlet of the second pump chamber and the third outlet port.

Preferably, the first inlet port is a clean dialysate solution inlet port, the second inlet port is a first dialysate solution constituent inlet port, the third inlet port is a second dialysate solution constituent inlet port and the fourth inlet port is a spent dialysate solution inlet port.

Preferably, the first outlet port is a clean dialysate solution outlet port to a first dialysate solution constituent, the second outlet port is a clean dialysate outlet port to the dialyser and the third outlet port is a drain port.

By way of example only a method of recirculating dialysate solution is described, the method comprising:
i) Providing a cartridge having a first inlet port, a conduit between the first inlet port and the inlet of the third pump chamber, a first outlet port, a conduit between the first inlet port and the first outlet port, a second inlet port, a conduit between the second inlet port and the inlet of the third pump chamber, a conduit between the outlet of the third pump chamber and the inlet of the fourth pump chamber, a third inlet port, a conduit between the third inlet port and the inlet of the fourth pump chamber, a conduit between the outlet of the fourth pump chamber and the inlet of the first pump chamber, a second outlet port, a conduit between the outlet of the first pump chamber and the second outlet port, a fourth inlet port, a conduit between the fourth inlet port and the inlet of the second pump chamber, a third outlet port, and, a conduit between the outlet of the second pump chamber and the third outlet port;
ii) Providing a dialyser having a housing defining a dialyser inlet and a dialyser outlet, the housing of the dialyser having a semi-permeable membrane therein separating blood passing longitudinally through the dialyser and a dialysate solution following a flow path between the inlet of the dialyser and the outlet of the dialyser, the inlet of the dialyser being connected to the second outlet port and the outlet of the dialyser being connected to the fourth inlet port
iii) Admitting a pre-mixed dialysate solution into the cartridge at the first inlet port
iv) Connecting the third outlet port to the first inlet port;
v) Disconnecting and blocking the third inlet port;
vi) Connecting the first outlet port to the second inlet port;
vii) Connecting the second outlet port to the inlet of the dialyser;
viii) Connecting the fourth inlet port to the outlet of the dialyser; and,
ix) Flowing dialysate solution through the hemodialysis machine at a fluid flow rate configured to saturate the semi-permeable membrane of the dialyser.

Advantageously, recirculating spent dialysate solution at a fluid flow rate configured to partially or fully saturate the semi-permeable membrane of the dialyser permits the dialysate solution to be circulated through the hemodialysis machine a number of times. This is useful, for example, when the patient is away from home and does not have access to either filtration equipment or a continuous supply of purified water.

By way of example only a hemodialysis machine defining a closed loop fluid path for dialysate solution is described, the hemodialysis machine comprising a mixing chamber for receiving dialysate solution, a first flow balance chamber connected to the mixing chamber, a second flow balance chamber connected to the first flow balance chamber, a dialyser for cleaning dialysate solution, the dialyser connected between the first flow balance chamber and the second flow balance chamber, and, a filter for cleaning dialysate solution, the filter connected between the second flow balance chamber and the mixing chamber, wherein the mixing chamber and first and second flow balance chambers are provided on a cartridge removably mountable in the hemodialysis machine.

By way of example only a hemodialysis machine defining a closed loop fluid path for dialysate solution is described, the hemodialysis machine comprising, a fixed volume of dialysate solution, a mixing chamber for receiving the dialysate solution, a first flow balance chamber connected to the mixing chamber, a second flow balance chamber connected between the first flow balance chamber and the mixing chamber, and, a dialyser connected between the first and second flow balance chambers, wherein, the mixing chamber, first flow balance chamber and second flow balance chamber are provided on a cartridge removably mountable in the hemodialysis machine.

By way of example only a hemodialysis machine comprising a cartridge removably mounted on the hemodialysis machine is described, the cartridge comprising a first flow balance pump chamber covered by a flexible diaphragm, the first flow balance pump chamber and flexible diaphragm defining a first pump, the first pump having a pump inlet and a pump outlet, a second flow balance pump chamber covered by a flexible diaphragm, the second flow balance pump chamber defining a second pump, the second pump having a pump inlet and a pump outlet, wherein each of the first and second pumps are provided with single fluid paths therethough between respective pump inlets and respective pump outlets.

Advantageously, the provision of a single flow path through each flow balance pump means that only two valves are required per pump to control the pass of liquid therethrough instead of four as used in prior art systems. Fewer valves and a single flow path is desirable to limit errors in flow balance control where measurement of fluid taken from a patient is required to be accurately measured.

An embodiment of the invention will now be described, by way of example only, with reference to the following figures.
Figure 1 shows a schematic of a dialysis system having a disposable cartridge comprising a fluid path defined by pumps and valves used for conventional single pass hemodialysis.
Figure 1a shows a detailed schematic view of the cartridge of Figure 1.
Figure 2 shows a schematic view of a system of verifying the stroke of a cartridge pump diaphragm of an embodiment of the present invention.
Figure 3 shows a schematic view of a flow balance valve arrangement in accordance with one embodiment of the invention.
Figure 4 shows a schematic side view of a flow balance pump according to an embodiment of the invention
Figure 5 shows a schematic view of a closed loop recirculating and regenerating dialysis system.
Figure 6 shows a schematic view of a closed loop recirculating dialysis system.

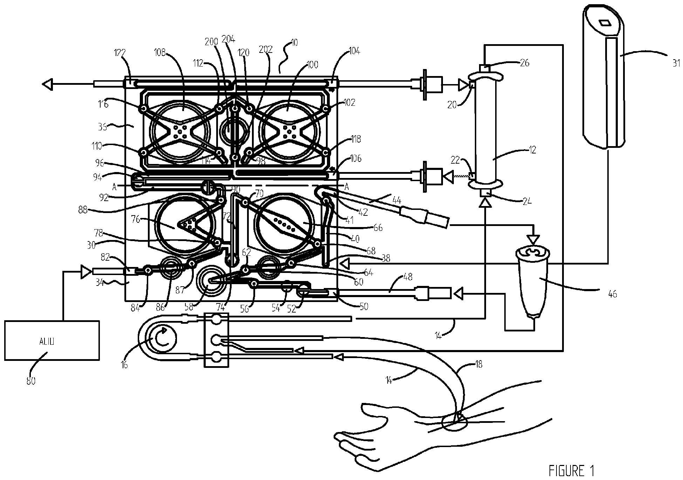

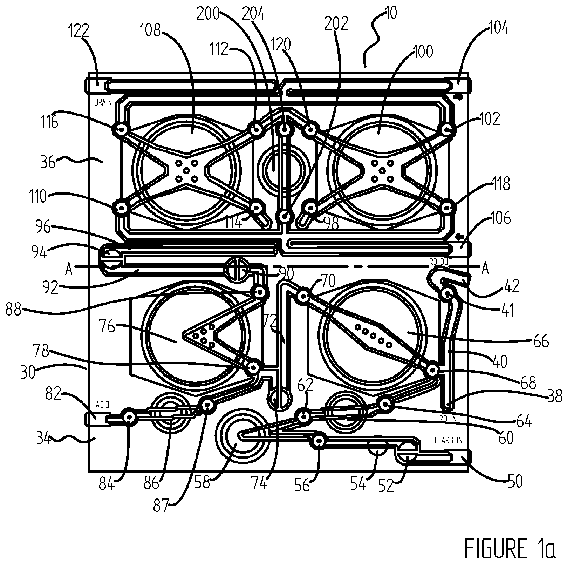

Referring to Figures 1 and 1a, a dialysis system, generally referred to as 10, is shown. A dialyser 12 receives blood via an arterial line 14 connected to a patient by a vascular access device (not shown for clarity), for example a hollow needle as typically used for drawing blood from a patient. The blood is pumped from the patient to the dialyser by a peristaltic pump 16. The blood passes through the dialyser in a known manner and is returned to the patient via a venous line 18. The dialyser 12 comprises a cylindrical tube closed by opposing ends. A semi-permeable membrane (not shown) is provided within the dialyser tube and separates the patients blood from a dialysate solution. The membrane extends substantially between the opposing ends of the cylinder. The dialysate solution removes impurities from the patients blood in a known manner.

The dialyser has an inlet 20 for receiving clean dialysate solution and an outlet 22 for removing spent dialysate solution from the dialyser 12. The dialyser also has an inlet 24 for receiving untreated blood from the peristaltic pump 16 and an outlet 26 for returning processed blood to the patient. The dialyser 12 is typically provided in a substantially upright orientation, in use, with the patients blood flowing longitudinally through the dialyser 12 from the blood inlet 24 to the blood outlet 26. The dialysate solution inlet 20 and dialysate solution outlet 22 are configured to be orientated substantially orthogonal to the blood inlet 24 and blood outlet 26, and to provide a counterflow. Dialysate solution is circulated through the hemodialysis machine at a fluid flow rate in the region of 400 to 800 ml/min for approximately four hours.

The dialysis system defines a fluid circuit including a cartridge 30 as will now be described. The cartridge 30 is a consumable component in the hemodialysis machine described.

The cartridge 30 is formed from an acrylic plastic such as SG-10 and has a machine side and a patient side. The cartridge 30 defines pump chambers which are closed by respective diaphragms, formed from, for example, DEHP-free PVC, to define respective pumps. In this embodiment, each diaphragm is part of a single, common sheet of material applied to the machine side of the cartridge 30. The individual diaphragms are operable by pneumatic pressure or vacuum applied thereto.

A series of flow paths are formed in the cartridge 30 for carrying dialysate solution constituted from water, bicarbonate solution and acid solution. The flow paths are located between the sheet of material closing the machine side of the cartridge 30 and a further sheet of the same material closing the patient side of the cartridge 30.

In use, the variation of pressure applied to the flexible diaphragm of each pump chamber is controlled by conventional valving. A pressure source applies either a positive or negative pressure to one side of the diaphragm of each pump chamber, as required, to pump fluid through the fluid paths in the cartridge 30, in a circuit defined by a plurality of valves.

The valves of the cartridge 30 are conventional diaphragm valves defined by respective openings in the cartridge 30 and closed by respective flexible diaphragms. Each valve is operable by applying a negative pressure to the diaphragm to open the valve and applying a positive pressure to the diaphragm to close the valve. The diaphragm of each valve is part of the single, common sheet of material applied to the machine side of the cartridge 30. The valves are opened and closed according to a flow control strategy, as will become apparent.

The machine side of the cartridge 30 abuts a pump driver (not shown) comprising a platen having a plurality of recessed surfaces, each recessed surface substantially corresponding in geometry and volume to a pump chamber defined in the cartridge 30. Each recessed surface has a fluid port connectable with a source of positive fluid pressure and, with a source of negative fluid pressure via a valve.

The positive and negative fluid pressure sources include a pressure pump and a vacuum pump respectively. When the valve is operated to allow fluid to flow into a recessed surface from the source of positive fluid pressure, the diaphragm moves into a corresponding pump chamber and any fluid, i.e. dialysate solution, therein is expelled from that pump chamber via the series of flow paths. When the valve is operated to allow fluid to flow out of a recessed surface to the source of negative fluid pressure, the diaphragm is moved away from a pump chamber and into the corresponding recessed surface to permit fluid to be drawn into that pump chamber via the series of flow paths. The surface of the pump chambers and of the platen provide a positive stop for each diaphragm, to prevent overstretching thereof. The positive stop ensures that the volume of fluid drawn into and pumped from the pump chambers is accurately controlled and remains constant during the dialysis session.

The cartridge 30 has two main functions, preparation of dialysate solution and flow balance. Each function is performed by a separate part of the cartridge as illustrated in Figures 1 and 1a by the schematic separation of the cartridge into two parts by the line A-A in the figures. The dialysate preparation function is performed by one part of the cartridge, generally referred to at 34 and the flow balance function is performed by the other part of the cartridge, generally referred to at 36. The cartridge 30 prepares an accurately mixed homogenous dialysate solution and ensures that the flow of clean dialysate supplied to the dialyser 12 matches (to within clinical tolerances) the volume of spent dialysate drawn from the dialyser 12.

The cartridge 30 is provided with a plurality of connections to and from the cartridge 30 as described below.

A first inlet port 38, from hereon referred to as the water inlet port, defined in the machine side of the cartridge 30 receives purified water from a purified water supply 31 such as a reverse osmosis water supply.

A first outlet port 42, from hereon referred to as the water outlet port, defined in an edge of the cartridge 30 directs the purified water to a first dialysate solution constituent which, in the illustrated embodiment shown in Figures 1 and 1a, is bicarbonate 46.

A second inlet port 50, from hereon referred to as the bicarbonate inlet port, defined in the same edge of the cartridge 30 as the water outlet port 42 receives purified water mixed with the bicarbonate 46.

A third inlet port 82, from hereon referred to as the acid inlet port, defined in the opposite edge of the cartridge 30 to the water outlet port 42 and bicarbonate inlet port 50 receives a second dialysate solution constituent which, in the illustrated embodiment shown in Figures 1 and 1a, is acid 80.

A second outlet port 104, from hereon referred to as the clean dialysate solution outlet port, is defined in the same edge of the cartridge as the water outlet port 42 and the bicarbonate inlet port 50. The clean dialysate outlet port 104 directs clean dialysate solution to the dialyser 12.

A fourth inlet port 106, from hereon referred to as the spent dialysate solution inlet port, is defined in the same edge of the cartridge 30 as the water outlet port 42, bicarbonate inlet port 50 and clean dialysate outlet port 104. The spent dialysate solution inlet port 106 receives spent dialysate solution from the dialyser 12.

A third outlet port 122, from hereon referred to as the drain port, is defined in the same edge of the cartridge as the acid inlet port 82. The drain port 122 directs spent dialysate solution out of the cartridge 30.

### Dialysate Preparation

Dialysate solution is prepared in the cartridge 30 by combining purified water with two dialysate constituents, namely a bicarbonate solution and an acid solution.

Purified water is admitted into the cartridge 30 from a purified water supply 31 via the water inlet port 38. The purified water passes through a channel 40 via a water inlet valve 41, when open, and exits the cartridge 30 at the water outlet port 42. From here, the purified water is carried by a tube 44 through a bicarbonate cartridge 46 in a known manner to generate a purified water and bicarbonate solution. The purified water and bicarbonate solution is carried by a tube 48 and re-admitted into the cartridge 30 via the bicarbonate inlet port 50.

The temperature of the bicarbonate solution is measured at sensing port 52 and the bicarbonate solution pressure is measured at sensing port 54. The bicarbonate solution passes a bicarbonate control valve 56, when open, before entering a bicarbonate solution reservoir 58 having an inlet and an outlet. The bicarbonate control valve 56 is closed when flow therethrough is not required.

A bicarbonate dosing pump chamber 60 having an inlet and an outlet receives the bicarbonate solution from the bicarbonate solution reservoir 58 through a bicarbonate dosing pump inlet valve 62. The bicarbonate dosing pump chamber 60 is closed by a diaphragm to define a bicarbonate dosing pump which, upon actuation of the diaphragm, pumps the bicarbonate solution from the bicarbonate dosing pump 60 to a first mixing pump chamber 66 (bicarbonate pump chamber). The bicarbonate dosing pump 60 has a bicarbonate dosing pump outlet valve 64 which is closed when the bicarbonate dosing pump inlet valve 62 is open. The bicarbonate dosing pump outlet valve 64 is opened to permit bicarbonate solution to be pumped to the bicarbonate pump chamber 66. When the bicarbonate dosing pump outlet valve 64 is open, the bicarbonate dosing pump inlet valve 62 is closed to prevent bicarbonate solution from being pumped back into the bicarbonate solution reservoir 58.

The bicarbonate pump chamber 66 having an inlet and an outlet receives the purified water and bicarbonate solution from the bicarbonate dosing pump 60 via a bicarbonate pump inlet valve 68. The bicarbonate pump inlet valve 68, when open, can also admit purified water into the bicarbonate pump chamber 66 from the water inlet port 38. The bicarbonate pump chamber 66 is closed by a diaphragm to define a pump which, upon actuation of the diaphragm, pumps the bicarbonate solution and purified water therein through a bicarbonate pump outlet valve 70 to a second mixing pump chamber 76 (acid pump).

When the bicarbonate pump inlet valve 68 is open, the bicarbonate pump outlet valve 70 and water outlet valve 41 are closed. When the bicarbonate pump outlet valve 70 is open, the bicarbonate pump inlet valve 68 is closed to prevent the bicarbonate and purified water solution from being pumped back into channel 40.

From the bicarbonate pump outlet valve 70, the bicarbonate and purified water solution enters a sensor channel 72 in which the hemodialysis machine measures the conductivity of the bicarbonate and purified water solution in a known manner. The bicarbonate and purified water solution then enters a temperature sensor 74 before, if the conductivity and temperature of the bicarbonate and purified water solution are within tolerance, entering the acid pump chamber 76.

The acid pump chamber 76 having an inlet and an outlet receives the bicarbonate and purified water solution from the bicarbonate pump 66 via an acid pump inlet valve 78. The acid pump inlet valve 78, when open, can also admit an acid solution into the pump chamber 76. The acid pump chamber 76 is closed by a diaphragm to define a pump which, upon actuation of the diaphragm, pumps the acid solution, bicarbonate solution and purified water therein through an acid pump outlet valve 88 to the first flow balance pump chamber 100. When the acid pump inlet valve 78 is open, the acid pump outlet valve 88 is closed. When the acid pump outlet valve 88 is open, the acid pump inlet valve 78 is closed.

The acid solution is admitted into the cartridge 30 from a pre-determined supply of acid 80 via the acid solution inlet port 82. From the acid solution inlet port the acid solution passes through an acid dosing pump chamber 86 via an acid dosing pump inlet valve 84 and an acid dosing pump outlet valve 87. The acid dosing pump outlet valve 87 is closed when the acid dosing pump inlet valve 84 is open. The acid dosing pump inlet valve 84 is closed when the acid dosing pump outlet valve 87 is open.

The dialysate solution exits the acid pump chamber via the acid pump outlet valve 88 and passes through a first dialysate solution temperature sensor 90 and a first dialysate solution conductivity sensor 92. A second dialysate solution temperature sensor 94 and a second dialysate solution conductivity sensor 96 are provided to corroborate the data provided by the first dialysate solution temperature sensor 90 and the first dialysate solution conductivity sensor 92. Providing the data measured by sensors 90, 92, 94 and 96 is within tolerance, the dialysate solution is admitted into a first flow balance pump chamber 100

### Flow Balance

The flow balance function of the cartridge 30 provides first and second flow balance pump chambers 100, 108, each having two inlets and two outlets to define two independent flow paths therethrough. The first and second flow balance pump chambers 100, 108 are of approximately equal volume. Either the first or second flow balance pump chamber 100, 108 pumps dialysate solution to a dialyser 12 and the other of the first or second flow balance pump chambers 100, 108 pumps dialysate solution from the dialyser 12 to the drain port 122. After every approximately 20 strokes of the first and second flow balance pumps 100, 108, their function is reversed.

From this point onwards, dialysate solution will be referred to as either clean dialysate solution or spent dialysate solution. Clean dialysate solution is intended to mean dialysate solution that is either new dialysate solution or clean dialysate solution that has been treated to remove waste product therefrom. Spent dialysate solution is intended to mean dialysate solution that has passed through the dialyser 12 to remove waste fluids from a patients blood into the dialysate solution.

Each of the first and second flow balance pump chambers 100, 108 are closed by a diaphragm to define respective pumps. The diaphragm is actuated away from a pump chamber by a negative pressure source to draw a volumetrically measured quantity of dialysate solution into the pump chamber. The diaphragm is actuated toward the pump chamber to pump the fluid therein out of an outlet.

The first flow balance pump chamber 100 has a clean dialysate solution inlet valve 98 for receiving clean dialysate solution from the acid pump 76 and a clean dialysate solution outlet valve 102 for pumping clean dialysate solution to the dialyser 12. The first flow balance pump chamber 100 also has a spent dialysate solution inlet valve 118 for receiving spent dialysate from the dialyser 12 and a spent dialysate solution outlet valve 120 for pumping the spent dialysate to drain via drain outlet port 122.

At any one time, only one of valves 98, 102, 118 or 120 will be open and the other three valves will be closed. The flow balance function, as described above, requires alternating the function of each flow balance pump approximately every 20 cycles. Therefore, when the first flow balance pump 100 is pumping clean dialysate solution to the dialyser 12, only valves 98 and 102 are in use and when the first flow balance pump 100 is pumping spent dialysate solution from the dialyser 12 to drain, only valves 118 and 120 will be in use.

The clean dialysate solution is pumped out of the first flow balance pump chamber 100 through the first flow balance pump clean dialysate solution outlet valve 102, upon closure of the first flow balance pump clean dialysate inlet valve 98, to the dialyser 12 via the dialyser outlet port 104.

Spent dialysate solution returns to the cartridge 30 from the dialyser 12 via the dialyser inlet port 106. The second flow balance pump chamber 108 has a spent dialysate solution inlet valve 110 for receiving spent dialysate solution from the dialyser 12 and a spent dialysate solution outlet valve 112 for pumping the spent dialysate solution to drain via drain outlet port 122. The second flow balance pump 108 also has a clean dialysate solution inlet valve 114 for receiving clean dialysate solution from the acid pump chamber 76 and a clean dialysate solution outlet valve 116 for pumping clean dialysate solution to the dialyser 12.

At any one time, only one of valves 110, 112, 114, 116 will be open and the other three valves will be closed. When the second flow balance pump 108 is pumping clean dialysate solution to the dialyser 12, only valves 114 and 116 will be in use and when the second flow balance pump 108 is pumping spent dialysate solution from the dialyser 12 to drain, only valves 114 and 116 will be in use.

In the illustrated example, the operation of the first and second flow balance pumps 100, 108 can be switched so that the first flow balance pump 100 is used to draw spent dialysate solution from the dialyser 12 and the second flow balance pump 108 is used to pump clean dialysate solution into the dialyser 12 as described below.

The clean dialysate solution is drawn into the second flow balance pump chamber 108 from the acid pump 76 via the second flow balance pump clean dialysate solution inlet valve 114 upon actuation of the diaphragm. The clean dialysate solution is then pumped from the second flow balance pump chamber 108 via the second flow balance pump clean dialysate solution outlet valve 116, upon closure of the clean dialysate solution inlet valve 114, to the dialyser 12.

Spent dialysate solution from the dialyser 12 is drawn into the first flow balance pump 100 via the second flow balance pump spent dialysate solution inlet valve 118. The spent dialysate solution is then pumped out of the first flow balance pump chamber 100 via the second flow balance pump spent dialysate solution outlet valve 120, upon closure of the spent dialysate solution inlet valve 118, to drain via drain outlet port 122.

The volume of fluid that is returned from the dialyser 12 is greater than the volume of fluid that is pumped to the dialyser via the first or second flow balance pump 100, 108. The first and second flow balance pumps have fixed volumes meaning that the excess fluid volume cannot be accommodated in the first or second flow balance pump. An ultrafiltration pump 200 is provided between the first and second flow balance pumps 100, 108 and has an inlet valve 202 and an outlet valve 204. The ultrafiltration pump 200 comprises a concave recess in the cartridge closed by a flexible diaphragm, the concave recess and the flexible diaphragm defining an ultrafiltration pump chamber.

In use, the inlet valve 202 of the ultrafiltration pump 200 is opened to allow the ultrafiltration pump 200 to draw in a pre-determined volume of spent dialysate solution. When the inlet valve 202 of the ultrafiltration pump 200 is open, the outlet valve 204 of the ultrafiltration pump 200 is closed. When the ultrafiltration pump 200 has received a volume of spent dialysate solution, the outlet valve 204 is opened and the spent dialysate solution in the ultrafiltration pump chamber is pumped through the outlet valve 204 to drain via the drain outlet port 122. When the outlet valve 204 of the ultrafiltration pump 200 is open, the inlet valve 202 of the ultrafiltration pump 200 is closed.

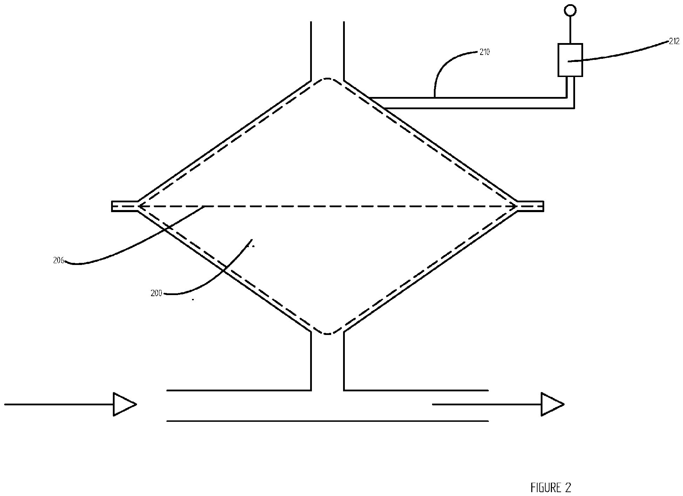

Figure 2 shows a system for measuring the volume of ultrafiltration drawn from a patients blood and discarded. Each full stroke of the ultrafiltration pump 200 draws a known volume of spent dialysate solution from the closed fluid flow path. To accurately measure the volume of fluid lost to the drain outlet port 122, each full stroke of the ultrafiltration pump 200 is counted. An optical sensor 212 is provided and connected to the ultrafiltration pump 200 by a flexible fibre optic cable 210 to detect whether the flexible diaphragm 206 of the ultrafiltration pump 200 is fully actuated on each stroke. If the sensor 212 detects that the flexible diaphragm 206 of the ultrafiltration pump 200 is not fully actuated, an alarm signal is sent to the hemodialysis machine and the fluid flow is stopped until the alarm signal is reset.

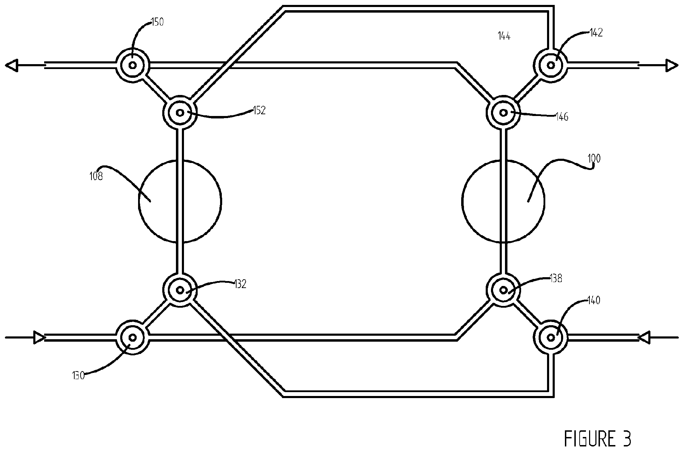

With reference to Figure 3, an alternative flow balance arrangement is shown. Clean dialysate solution is drawn from the acid pump 76 into the first flow balance pump chamber 100 via valves 130 and 138. The clean dialysate solution is pumped from the first flow balance pump chamber 100, upon closure of valve 138, to the dialyser 12 via valves 146 and 142. Spent dialysate solution is drawn into the second flow balance pump 108 from the dialyser 12 via valves 140 and 132. The second flow balance pump 108 pumps the spent dialysate solution via valves 152 and 150, upon closure of valve 132, to exit the cartridge 30 via the drain port 122.

Similar to the example as described with reference to Figure 1, the function of the first and second flow balance pumps 100, 108 can be swapped so that the first flow balance pump 100 is used to draw spent dialysate solution from the dialyser 12 and the second flow balance pump 108 is used to pump clean dialysate solution into the dialyser 12 as described below.

Clean dialysate solution is drawn into the second flow balance chamber 108 via valves 130 and 132. The clean dialysate solution is then pumped to the dialyser 12 via valves 152 and 142, upon closure of valve 132. Spent dialysate solution is then drawn into the first flow balance pump chamber 100 via valves 140 and 138. The first flow balance pump 100 pumps the spent dialysate solution via valves 146 and 150, upon closure of valve 138, to exit the cartridge 30 via the drain port 122.

Each flow balance pump 100, 108 has a single fluid path therethrough with a valve at each end of each fluid path. Valves 130, 134, 136, 140, 142, 144, 148 and 150 are not directly associated with the flow balance pumps 100, 108 and one or more of said valves can be bypassed in some embodiments of the invention.

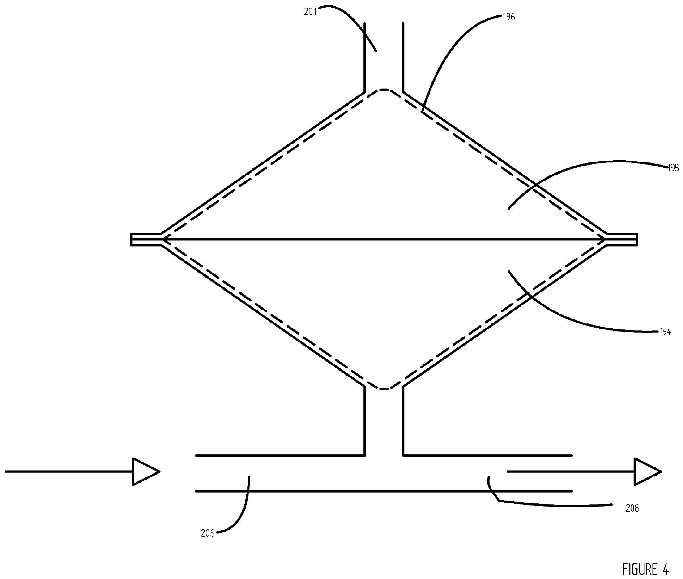

Figure 4 shows a representative view of a flow balance pump 100 according to the present invention. The flow balance pump chamber 194 is provided on the cartridge and is closed by a diaphragm 196 which, at rest, sits across the pump chamber 194. The pump chamber receives either clean or spent dialysate solution via a dialysate solution inlet port 206 and pumps dialysate solution from the pump chamber via a dialysate solution outlet port 208.

The cartridge 30 is removably mounted into a hemodialysis machine which has a flow balance pump cavity 198 substantially corresponding in dimension and shape to the pump chamber 194. Upon supply of positive or negative pressure via a pump cavity pressure inlet port 201, the diaphragm is actuated into either the pump chamber 194 or pump cavity 198 to either draw fluid into the pump chamber 194 or pump fluid from the pump chamber 194.

### Regeneration

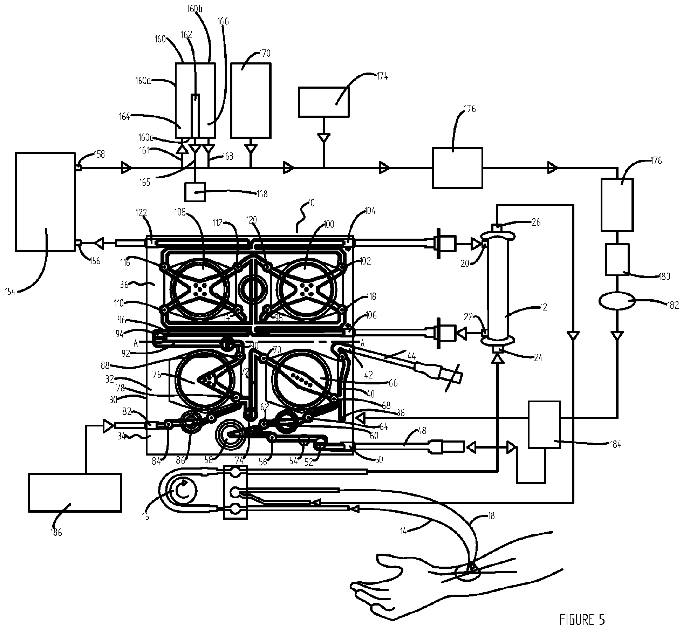

In Figures 1 and 1a, the spent dialysis is shown as being pumped to drain and therefore discarded. Figure 5, as described below, illustrates a system for regenerating and recirculating spent dialysis.

At the beginning of a dialysis session, the hemodialysis machine is primed with a known quantity of pre-mixed dialysate solution or purified water via flow input ports 170 or 174. The input port(s) 170, 174 for the pre-mixed dialysate solution and purified water are located at an elevation higher than the highest elevation of the hemodialysis machine.

If purified water is used to prime the hemodialysis machine, the purified water is circulated through the hemodialysis machine prior to connection to the patient to dose the purified water in a known manner by circulating the purified water through a dialysate regeneration device.

Instead of spent dialysate solution being pumped to drain via the drain outlet port 122, Figure 5 shows that spent dialysate solution is pumped to the dialysate regeneration device cartridge 154 via the drain outlet port 122.

The dialysate regeneration device 154 in the illustrated example comprises a material, such as sorbent, within a container that has a spent dialysate solution inlet 156 and a clean dialysate solution outlet 158. The spent dialysate solution is pumped through the sorbent to regenerate the spent dialysate solution in a known manner.

"Ultrafiltrate" is intended to mean the waste product which is removed from a patients blood into the dialysate solution during hemodialysis treatment.

The dialysate regeneration device 154 sets the proportion of the base constituent parts, i.e. acid and bicarbonate, in the dialysate solution after removal of ultrafiltrate from the dialysate solution. The clean dialysate solution, after regeneration, is of greater volume in the system than at the beginning of the dialysis session. The excess liquid in the system is removed via an overflow device 162 provided in a reservoir 160.

The reservoir 160 comprises a housing defining a continuous sidewall 160a, a bottom 160b, a top 160c, a reservoir inlet 161 and a reservoir outlet 163. The overflow device 162 is connected to the reservoir bottom 160b at a first end 162a and at a second end 162b defines a gap between said second end 162b and the top 160b of the reservoir 160. The overflow device 162 comprises a hollow tube that, when excess liquid is present in the system, directs that excess liquid into a reservoir drain port 165 ensuring that a consistent volume of liquid is maintained in the hemodialysis system, to the exclusion of air.

Sodium bicarbonate is a natural food source for biofilm which can cause bio-incompatibility and unacceptable errors in clean dialysate solution dosing and control. An endotoxin filter 176 is provided downstream of the reservoir 160 for removing biofilm flushed through the clean dialysate solution fluid circuit by the dialysate solution.

The clean dialysate solution having been filtered and potentially stored in the reservoir for a period of time is likely to be at a temperature below that of the human body. A heater 178 is provided downstream of the endotoxin filter 176 for heating the clean dialysate solution to a temperature of about 37°C before being passed through an air vent 180 to remove any bubbles from the clean dialysate solution.

The clean dialysate solution passes through a conventional ammonium sensor 182 for detecting the presence of ammonium in the dialysate solution before it enters the cartridge 30 via the water inlet port 38. The clean dialysate solution follows the fluid circuit back out of the cartridge 30, through the water outlet port 42, and passes through a dilution water supply 184 before re-entering the cartridge 30 via the bicarbonate inlet port 50. The dilution water supply 184 is used to adjust the conductivity of the dialysate solution on each pass through the cartridge 30.

The acid supply is replaced with a sodium chloride supply 186 for adjusting the conductivity of the clean dialysate solution on each pass of the dialysate solution through the cartridge 30 in accordance with a measurement taken by conductivity sensor 54. The acid dosing pump 86 pumps sodium chloride to the acid pump 76 which in turn pumps homogenous, clean dialysate solution to the first flow balance pump 100. The flow balance pumps 100, 108 operate as described previously.

### Dialysate Solution Recirculation

In situations where neither filtration equipment nor a continuous purified water supply are available, a single pre-mixed supply of clean dialysate solution can be re-circulated through the hemodialysis machine. By re-circulating the dialysate solution at a comparatively slow speed, i.e. between 100ml/min and 300ml/min, configured to saturate the semi-permeable diaphragm of the dialyser 12 with dialysate solution, the dialysate solution remains viable for a number of cycles through the hemodialysis machine.

A pre-mixed supply of dialysate solution is provided in a vessel.

In one embodiment, the dialysate solution is circulated through the hemodialysis machine at 100ml/min and saturates the semi-permeable membrane of the dialyser within one pass of dialysate solution through the hemodialysis machine. The vessel holds a volume of dialysate solution in the order of 60 litres with approximately 25 litres of dialysate solution being circulated through the hemodialysis machine in a single dialysis session.

In another embodiment, the dialysate solution is circulated through the hemodialysis machine at 200 to 300ml/min and saturates the semi-permeable membrane of the dialyser after a number of passes of dialysate solution through the hemodialysis machine. The vessel holds a volume of pre-mixed dialysate solution in the order of 20 litres for approximately two hours. The dialysate solution is re-circulated through the hemodialysis machine a number of times.

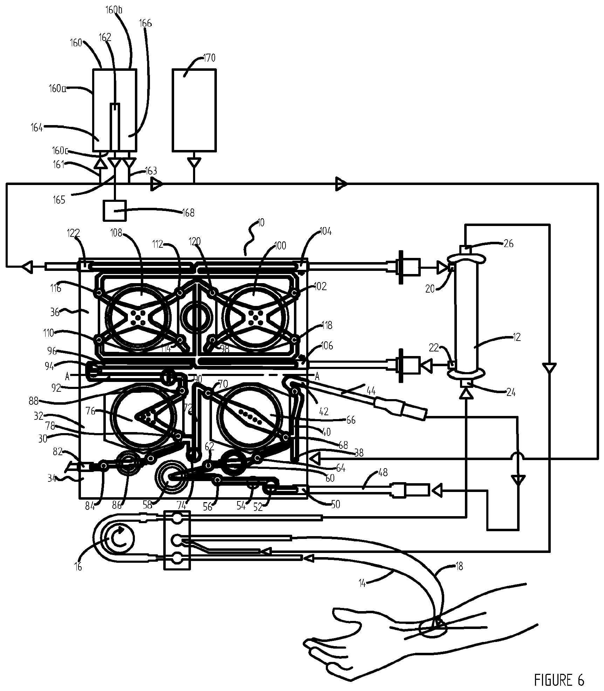

Referring to Figure 6, the dialysate solution can be re-circulated using the following method:
i) Connecting the drain port 122 to the clean dialysate inlet port 38;
ii) Disconnecting and blocking the acid inlet port 82;
iii) Connecting the water outlet port 42 to the bicarbonate inlet port 50;
iv) Priming the hemodialysis machine with pre-mixed clean dialysate solution and purified water;
v) Connecting the clean dialysate outlet port to the inlet of the dialyser;
vi) Connecting the outlet of the dialyser to the spent dialysate solution inlet port and,
v) Flowing dialysate solution through the hemodialysis machine 10 at a fluid flow rate configured to saturate the semi-permeable diaphragm of the dialyser 12.

The reservoir, as described with reference to Figure 5, takes ultrafiltrate generated during a dialysis session and removes it from the system via the reservoir overflow device 162 to the reservoir drain 168.

The embodiments of the present invention, described with reference to the figures, are examples only and do not exclude variations therefrom from the scope of the invention as defined by the claims.

## Claims

1. A hemodialysis machine defining a closed loop fluid path for dialysate solution, the fluid path comprising:
a first pump chamber having a pump inlet and a pump outlet;
a dialyser (12) having a dialyser inlet (20) and a dialyser outlet (22);
a conduit connected between the outlet of the first pump chamber and the inlet of the dialyser;
a second pump chamber having a pump inlet and a pump outlet;
a conduit connected between the outlet of the dialyser (12) and the inlet of the second pump chamber;
a dialysate regeneration device (154) having an inlet (156) and an outlet (158);
a conduit connected between the outlet of the second pump chamber and the inlet of the dialysate regeneration device;
a reservoir (160) having a reservoir inlet (161) and a reservoir outlet (163);
a conduit connected between the outlet of the dialysate regeneration device (154) and the inlet of the reservoir (160); and,
a conduit connected between the outlet of the reservoir (160) and the inlet of the first pump chamber, **characterized in that** the reservoir (160) comprises
a housing defining a continuous side wall (160a), a top (160c), a bottom (160b), the inlet (161) of the reservoir (160) and the outlet (163) of the reservoir (160), and an overflow device (162) mounted therein.

2. A hemodialysis machine according to Claim 1 further comprising:
a third pump chamber having a pump inlet and a pump outlet, the third pump chamber being connected between the reservoir (160) and the first pump chamber inlet, preferably, the hemodialysis machine further comprises a fourth pump chamber having a pump inlet and a pump outlet, the fourth pump chamber being connected between the third pump chamber and the first pump chamber, preferably still, the hemodialysis machine further comprises a fifth pump chamber having a pump inlet and a pump outlet, the fifth pump chamber being connected between the first pump chamber and the second pump chamber.

3. A hemodialysis machine according to Claim 2, wherein the first, second, third, fourth and fifth pump chambers are closed by a flexible diaphragm defining respective first, second, third, fourth and fifth pumps.

4. A hemodialysis machine according to Claim 3, wherein the first and second pumps are flow balance pumps (100, 108).

5. A hemodialysis machine according to any preceding claim, wherein the dialysate regeneration device (154) comprises a housing, the housing defining the inlet (156) of the dialysate regeneration device (154) and the outlet (158) of the dialysate regeneration device (154), the housing further defining a fluid path therethrough between the inlet (156) of the dialysate regeneration device (154) and the outlet (158) of the dialysate regeneration device (154).

6. A hemodialysis machine according to Claim 1, wherein the overflow device (162) comprises a hollow tube having an inlet and an outlet, the overflow device (162) having a first end connected to the bottom of the reservoir (160) and a second end defining a gap between the top of the reservoir (160) and second end, wherein dialysate solution in the reservoir (160) exceeding a pre-determined volume set by the gap between the second end of the overflow device (162) and the top of the reservoir (160) is admitted into the overflow device (162).

7. A hemodialysis machine according to Claim 1, wherein dialysate solution admitted into the overflow device (162) is expelled from the reservoir (160) through a reservoir drain outlet (165).

8. A hemodialysis machine according to any of Claims 2 to 7, wherein each of the first, second, third, fourth and fifth pump chambers are provided on a cartridge releasably mountable in the hemodialysis machine, preferably wherein the cartridge (130) is disposable.

9. A hemodialysis machine according to Claim 8, wherein the cartridge (130) further comprises:
a first inlet port;
a conduit between the first inlet port and the inlet of the third pump chamber;
a first outlet port;
a conduit between the first inlet port and the first outlet port;
a second inlet port;
a conduit between the second inlet port and the inlet of the third pump chamber;
a conduit between the outlet of the third pump chamber and the inlet of the fourth pump chamber;
a third inlet port;
a conduit between the third inlet port and the inlet of the fourth pump chamber;
a conduit between the outlet of the fourth pump chamber and the inlet of the first pump chamber;
a second outlet port;
a conduit between the outlet of the first pump chamber and the second outlet port;
a fourth inlet port;
a conduit between the fourth inlet port and the inlet of the second pump chamber;
a third outlet port; and,
a conduit between the outlet of the second pump chamber and the third outlet port.

10. A hemodialysis machine according to Claim 9, wherein the cartridge (130) further comprises:
a conduit between the second pump chamber and the fifth pump chamber, and
a conduit between the fifth pump chamber and the drain outlet, preferably wherein the first inlet port is a clean dialysate solution inlet port, the second inlet port is a first dialysate solution constituent inlet port, the third inlet port is a second dialysate solution constituent inlet port and the fourth inlet port is a spent dialysate solution inlet port, preferably still, wherein the first outlet port is a first dialysate solution constituent outlet port, the second outlet port is a clean dialysate outlet port to the dialyser and the third outlet port is a drain port.

11. A hemodialysis machine according to any preceding claim further comprising a water inlet port, an endotoxin filter (176) and a heater (178) downstream of the reservoir (160), preferably, wherein the endotoxin filter (176) is downstream of the water inlet port, preferably still, wherein the heater (178) is downstream of the endotoxin filter (176).

12. A hemodialysis machine according to Claim 11 further comprising an air vent and ammonium sensor (182) downstream of the heater, preferably wherein the ammonium sensor (182) is downstream of the air vent.

13. A hemodialysis machine according to any of Claims 2 to 12, wherein the first, second, third, fourth and fifth pump chambers are concave.

14. A hemodialysis machine according to any of Claims 10 to 12 further comprising a purified water inlet port between the reservoir and the endotoxin (176) filter.

15. A hemodialysis machine according to any preceding claim wherein the dialysate regeneration device (154) comprises a sorbent material, preferably, wherein the hemodialysis machine further comprises a light sensor for measuring and verifying the stroke position of a pump chamber diaphragm, preferably still, wherein the dialyser and dialysate regeneration device are connected to opposing sides of the cartridge (130).

## Patentansprüche

1. Hämodialysegerät, das einen Fluidpfad mit geschlossenem Kreislauf für eine Dialysatlösung definiert, wobei der Fluidpfad umfasst:
eine erste Pumpenkammer mit einem Pumpeneinlass und einem Pumpenauslass;
einen Dialysator (12) mit einem Dialysatoreinlass (20) und einem Dialysatorauslass (22);
eine Leitung, die zwischen dem Auslass der ersten Pumpenkammer und dem Einlass des Dialysators angeschlossen ist;
eine zweite Pumpenkammer mit einem Pumpeneinlass und einem Pumpenauslass;
eine Leitung, die zwischen dem Auslass des Dialysators (12) und dem Einlass der zweiten Pumpenkammer angeschlossen ist;
eine Dialysatregenerationseinrichtung (154) mit einem Einlass (156) und einem Auslass (158);
eine Leitung, die zwischen dem Auslass der zweiten Pumpenkammer und dem Einlass der Dialysatregenerationseinrichtung angeschlossen ist;
ein Reservoir (160) mit einem Reservoireinlass (161) und einem Reservoirauslass (162);
eine Leitung, die zwischen dem Auslass der Dialysatregenerationseinrichtung (154) und dem Einlass des Reservoirs (160) angeschlossen ist; und
eine Leitung, die zwischen dem Auslass des Reservoirs (160) und dem Einlass der ersten Pumpenkammer angeschlossen ist,
**dadurch gekennzeichnet, dass** das Reservoir (160) ein Gehäuse umfasst, welches eine durchgehende Seitenwand (160a), eine Oberseite (160c), einen Boden (160b), den Einlass (161) des Reservoirs (160) und den Auslass (163) des Reservoirs (160) sowie eine darin angeordnete Überlaufeinrichtung (162) definiert.

2. Hämodialysegerät nach Anspruch 1, ferner umfassend:
eine dritte Pumpenkammer mit einem Pumpeneinlass und einem Pumpenauslass, wobei die dritte Pumpenkammer zwischen dem Reservoir (160) und dem Einlass der ersten Pumpenkammer angeschlossen ist, wobei vorzugsweise das Hämodialysegerät des Weiteren eine vierte Pumpenkammer mit einem Pumpeneinlass und einem Pumpenauslass umfasst, wobei die vierte Pumpenkammer zwischen der dritten Pumpenkammer und der ersten Pumpenkammer angeschlossen ist, wobei weiter vorzugsweise das Hämodialysegerät des Weiteren eine fünfte Pumpenkammer mit einem Pumpeneinlass und einem Pumpenauslass umfasst, wobei die fünfte Pumpenkammer zwischen der ersten Pumpenkammer und der zweiten Pumpenkammer angeschlossen ist.

3. Hämodialysegerät nach Anspruch 2, wobei die erste, zweite, dritte, vierte und fünfte Pumpenkammer durch eine flexible Membran verschlossen sind, welche die jeweilige erste, zweite, dritte, vierte und fünfte Pumpe definiert.

4. Hämodialysegerät nach Anspruch 3, wobei die erste und die zweite Pumpe Flussausgleichspumpen (100, 108) sind.

5. Hämodialysegerät nach einem der vorhergehenden Ansprüche, bei dem die Dialysatregenerationseinrichtung (154) ein Gehäuse aufweist, wobei das Gehäuse den Einlass (156) der Dialysatregenerationseinrichtung (154) und den Auslass (158) der Dialysatregenerationseinrichtung (154) definiert, wobei das Gehäuse ferner einen Fluidpfad zwischen dem Einlass (156) der Dialysatregenerationseinrichtung (154) und dem Auslass der Dialysatregenerationseinrichtung (154) definiert.

6. Hämodialysegerät nach Anspruch 1, wobei die Überlaufeinrichtung (162) ein hohles Rohr mit einem Einlass und einem Auslass umfasst, wobei die Überlaufeinrichtung (162) ein erstes, am Boden des Reservoirs (160) angeschlossenes Ende, und ein zweites Ende, das einen Spalt zwischen der Oberseite des Reservoirs (160) und dem zweiten Ende definiert, aufweist, wobei Dialysatlösung in dem Reservoir (160), die ein vorbestimmtes, durch den Spalt zwischen dem zweiten Ende der Überlaufeinrichtung (162) und der Oberseite des Reservoirs (160) festgelegtes Volumen überschreitet, in die Überlaufeinrichtung (162) eingelassen wird.

7. Hämodialysegerät nach Anspruch 1, wobei in die Überlaufvorrichtung (162) eingelassene Dialysatlösung aus dem Reservoir (160) durch einen Reservoirablaufauslass (165) ausgetrieben wird.

8. Hämodialysegerät nach einem der Ansprüche 2 bis 7, wobei jede der ersten, zweiten, dritten, vierten und fünften Pumpenkammern an einer Kartusche vorgesehen ist, die lösbar in dem Hämodialysegerät montierbar ist, wobei vorzugsweise die Kartusche (130) wegwerfbar ist.

9. Hämodialysegerät nach Anspruch 8, wobei die Kartusche (130) ferner Folgendes umfasst:
eine erste Einlassöffnung,
eine Leitung zwischen der ersten Einlassöffnung und dem Einlass der dritten Pumpenkammer,
eine erste Auslassöffnung,
eine Leitung zwischen der ersten Einlassöffnung und der ersten Auslassöffnung,
eine zweite Einlassöffnung,
eine Leitung zwischen der zweiten Einlassöffnung und dem Einlass der dritten Pumpenkammer,
eine Leitung zwischen dem Auslass der dritten Pumpenkammer und dem Einlass der vierten Pumpenkammer,
eine dritte Einlassöffnung,
eine Leitung zwischen der dritten Einlassöffnung und dem Einlass der vierten Pumpenkammer,
eine Leitung zwischen dem Auslass der vierten Pumpenkammer und dem Einlass der ersten Pumpenkammer,
eine zweite Auslassöffnung,
eine Leitung zwischen dem Auslass der ersten Pumpenkammer und der zweiten Auslassöffnung,
eine vierte Einlassöffnung,
eine Leitung zwischen der vierten Einlassöffnung und dem Einlass der zweiten Pumpenkammer,
eine dritte Auslassöffnung, und
eine Leitung zwischen dem Auslass der zweiten Pumpenkammer und der dritten Auslassöffnung.

10. Hämodialysegerät nach Anspruch 9, bei dem die Kartusche (130) ferner aufweist:
eine Leitung zwischen der zweiten Pumpenkammer und der fünften Pumpenkammer und
eine Leitung zwischen der fünften Pumpenkammer und dem Abflussauslass, wobei vorzugsweise die erste Einlassöffnung eine Einlassöffnung für saubere Dialysatlösung ist, die zweite Einlassöffnung eine Einlassöffnung für einen ersten Dialysatlösungsbestandteil ist, die dritte Einlassöffnung eine Einlassöffnung für einen zweiten Dialysatlösungsbestandteil ist und die vierte Einlassöffnung eine Einlassöffnung für verbrauchte Dialysatlösung ist, wobei weiterhin vorzugsweise die erste Auslassöffnung eine Auslassöffnung für den ersten Dialysatlösungsbestandteil ist, die zweite Auslassöffnung eine Austrittsöffnung für sauberes Dialysat zum Dialysator ist und die dritte Auslassöffnung eine Abflussöffnung ist.

11. Hämodialysegerät nach einem der vorhergehenden Ansprüche, ferner mit einer Wassereinlassöffnung, einem Endotoxinfilter (176) und einem Heizgerät (178) stromabwärts des Reservoirs (160), wobei vorzugsweise der Endotoxinfilter (176) stromabwärts zur Wassereinlassöffnung angeordnet ist, weiter vorzugsweise wobei das Heizgerät (178) stromabwärts zum Endotoxinfilter (176) angeordnet ist.

12. Hämodialysegerät nach Anspruch 11, des Weiteren umfassend eine Entlüftung und einen Ammoniumsensor (182) stromabwärts der Heizeinrichtung, wobei vorzugsweise der Ammoniumsensor (182) stromabwärts der Entlüftung angeordnet ist.

13. Hämodialysegerät nach einem der Ansprüche 2 bis 12, wobei die erste, zweite, dritte, vierte und fünfte Pumpenkammer konkav sind.

14. Hämodialysegerät nach einem der Ansprüche 10 bis 12, ferner umfassend eine Einlassöffnung für gereinigtes Wasser zwischen dem Reservoir und dem Endotoxinfilter (176).

15. Hämodialysegerät nach einem der vorhergehenden Ansprüche, wobei die Dialysatregenerationseinrichtung (154) ein Sorptionsmaterial umfasst, wobei vorzugsweise das Hämodialysegerät ferner einen Lichtsensor zum Messen und Überprüfen der Hubposition einer Pumpenkammermembran umfasst, wobei weiter vorzugsweise der Dialysator und die Dialysatregenerationseinrichtung mit gegenüberliegenden Seiten der Kartusche (130) verbunden sind.

## Revendications

1. Machine d'hémodialyse définissant une trajectoire de fluide en boucle fermée pour la solution de dialysat, la trajectoire de fluide comprenant :
une première chambre de pompe ayant une entrée de pompe et une sortie de pompe ;
un dialyseur (12) ayant une entrée de dialyseur (20) et une sortie de dialyseur (22) ;
un conduit raccordé entre la sortie de la première chambre de pompe et l'entrée du dialyseur ;
une deuxième chambre de pompe ayant une entrée de pompe et une sortie de pompe ;
un conduit raccordé entre la sortie du dialyseur (12) et l'entrée de la deuxième chambre de pompe ;
un dispositif de régénération de dialysat (154) ayant une entrée (156) et une sortie (158) ;
un conduit raccordé entre la sortie de la deuxième chambre de pompe et l'entrée du dispositif de régénération de dialysat ;
un réservoir (160) ayant une entrée de réservoir (161) et une sortie de réservoir (163) ;
un conduit raccordé entre la sortie du dispositif de régénération de dialysat (154) et l'entrée du réservoir (160) ; et
un conduit raccordé entre la sortie du réservoir (160) et l'entrée de la première chambre de pompe, **caractérisée en ce que** le réservoir (160) comprend :
un boîtier définissant une paroi latérale continue (160a), une partie supérieure (160c), une partie inférieure (160b), l'entrée (161) du réservoir (160) et la sortie (163) du réservoir (160), et un dispositif de trop-plein (162) monté à l'intérieur de ce dernier.

2. Machine d'hémodialyse selon la revendication 1, comprenant en outre :
une troisième chambre de pompe ayant une entrée de pompe et une sortie de pompe, la troisième chambre de pompe étant raccordée entre le réservoir (160) et l'entrée de la première chambre de pompe, de préférence la machine d'hémodialyse comprend en outre une quatrième chambre de pompe ayant une entrée de pompe et une sortie de pompe, la quatrième chambre de pompe étant raccordée entre la troisième chambre de pompe et la première chambre de pompe, encore de préférence la machine d'hémodialyse comprend en outre une cinquième chambre de pompe ayant une entrée de pompe et une sortie de pompe, la cinquième chambre de pompe étant raccordée entre la première chambre de pompe et la deuxième chambre de pompe.

3. Machine d'hémodialyse selon la revendication 2, dans laquelle les première, deuxième, troisième, quatrième et cinquième chambres de pompe sont fermées par un diaphragme flexible définissant les première, deuxième, troisième, quatrième et cinquième pompes respectives.

4. Machine d'hémodialyse selon la revendication 3, dans laquelle les première et deuxième pompes sont des pompes de régularisation de débit (100, 108).

5. Machine d'hémodialyse selon l'une quelconque des revendications précédentes, dans laquelle le dispositif de régénération de dialysat (154) comprend un boîtier, le boîtier définissant l'entrée (156) du dispositif de régénération de dialysat (154) et la sortie (158) du dispositif de régénération de dialysat (154), le boîtier définissant en outre une trajectoire de fluide à travers ce dernier entre l'entrée (156) du dispositif de régénération de dialysat (154) et la sortie (158) du dispositif de régénération de dialysat (154).

6. Machine d'hémodialyse selon la revendication 1, dans laquelle le dispositif de trop-plein (162) comprend un tube creux ayant une entrée et une sortie, le dispositif de trop-plein (162) ayant une première extrémité raccordée au fond du réservoir (160) et une seconde extrémité définissant un espace entre la partie supérieure du réservoir (160) et la seconde extrémité, dans laquelle la solution de dialysat dans le réservoir (160) dépassant un volume prédéterminé, déterminé par l'espace entre la seconde extrémité du dispositif de trop-plein (162) et la partie supérieure du réservoir (160) est admise dans le dispositif de trop-plein (162).

7. Machine d'hémodialyse selon la revendication 1, dans laquelle la solution de dialysat admise dans le dispositif de trop-plein (162) est expulsée du réservoir (160) par une sortie de drain de réservoir (165).

8. Machine d'hémodialyse selon l'une quelconque des revendications 2 à 7, dans laquelle chacune des première, deuxième, troisième, quatrième et cinquième chambres de pompe est prévue sur une cartouche pouvant être montée de manière amovible dans la machine d'hémodialyse, de préférence dans laquelle la cartouche (130) est jetable.

9. Machine d'hémodialyse selon la revendication 8, dans laquelle la cartouche (130) comprend en outre :
un premier orifice d'entrée ;
un conduit entre le premier orifice d'entrée et l'entrée de la troisième chambre de pompe ;
un premier orifice de sortie ;
un conduit entre le premier orifice d'entrée et le premier orifice de sortie ;
un deuxième orifice d'entrée ;
un conduit entre le deuxième orifice d'entrée et l'entrée de la troisième chambre de pompe ;
un conduit entre la sortie de la troisième chambre de pompe et l'entrée de la quatrième chambre de pompe ;
un troisième orifice d'entrée ;
un conduit entre le troisième orifice d'entrée et l'entrée de la quatrième chambre de pompe ;
un conduit entre la sortie de la quatrième chambre de pompe et l'entrée de la première chambre de pompe ;
un deuxième orifice de sortie ;
un conduit entre la sortie de la première chambre de pompe et le deuxième orifice de sortie ;
un quatrième orifice d'entrée ;
un conduit entre le quatrième orifice d'entrée et l'entrée de la deuxième chambre de pompe ;
un troisième orifice de sortie ; et
un conduit entre la sortie de la deuxième chambre de pompe et le troisième orifice de sortie.

10. Machine d'hémodialyse selon la revendication 9, dans laquelle la cartouche (130) comprend en outre :
un conduit entre la deuxième chambre de pompe et la cinquième chambre de pompe, et
un conduit entre la cinquième chambre de pompe et la sortie de drain, de préférence dans laquelle le premier orifice d'entrée est un orifice d'entrée de solution de dialysat propre, le deuxième orifice d'entrée est un premier orifice d'entrée de constituant de solution de dialysat, le troisième orifice d'entrée est un deuxième orifice d'entrée de constituant de solution de dialysat et le quatrième orifice d'entrée est un orifice d'entrée de solution de dialysat usagée, encore de préférence, dans laquelle le premier orifice de sortie est un premier orifice de sortie de constituant de solution de dialysat, le deuxième orifice de sortie est un orifice de sortie de dialysat propre vers le dialyseur, et le troisième orifice de sortie est un orifice de drain.

11. Machine d'hémodialyse selon l'une quelconque des revendications précédentes, comprenant en outre un orifice d'entrée d'eau, un filtre d'endotoxine (176) et un dispositif de chauffage (178) en aval du réservoir (160), de préférence, dans laquelle le filtre d'endotoxine (176) est en aval de l'orifice d'entrée d'eau, encore de préférence, dans lequel le dispositif de chauffage (178) est en aval du filtre d'endotoxine (176).

12. Machine d'hémodialyse selon la revendication 11, comprenant en outre un évent d'air et un capteur d'ammoniaque (182) en aval du dispositif de chauffage, de préférence dans laquelle le capteur d'ammoniaque (182) est en aval de l'évent d'air.

13. Machine d'hémodialyse selon l'une quelconque des revendications 2 à 12, dans laquelle les première, deuxième, troisième, quatrième et cinquième chambres de pompe sont concaves.

14. Machine d'hémodialyse selon l'une quelconque des revendications 10 à 12, comprenant en outre un orifice d'entrée d'eau purifiée entre le réservoir et le filtre d'endotoxine (176).

15. Machine d'hémodialyse selon l'une quelconque des revendications précédentes, dans laquelle le dispositif de régénération de dialysat (154) comprend un matériau sorbant, de préférence, dans laquelle la machine d'hémodialyse comprend en outre un capteur de lumière pour mesurer et vérifier la position de course d'un diaphragme de chambre de pompe, encore de préférence, dans laquelle le dialyseur et le dispositif de régénération de dialysat sont raccordés aux côtés opposés de la cartouche (130).
